# EUROPEAN PATENT SPECIFICATION

(11) **EP 0 941 036 B1**
(45) Date of publication and mention of the grant of the patent: **03.11.2004**
(21) Application number: 98949670.8
(22) Date of filing: 30.09.1998
(51) Int. Cl.: A61B 17/22

(54) **MEDICAL RETRIEVAL BASKET WITH LEGS SHAPED TO ENHANCE CAPTURE SOLID MATTER AND REDUCE TRAUMA**
MEDIZINISCHES FANGKÖRBCHEN MIT GREIFARMEN, IN DER ART GESTALTET, DASS DAS EINFANGEN VON FESTEN TEILCHEN VERBESSERT UND DAS TRAUMA VERMINDERT WIRD
PANIER D'EXTRACTION MEDICAL MUNI DE BRAS FORMES POUR FACILITER LA PREHENSION DE MATIERES SOLIDES ET REDUIRE LES TRAUMATISMES

(30) Priority: 01.10.1997 US 60830 P; 01.10.1997 US 60819 P; 01.10.1997 US 60821 P; 23.02.1998 US 27534; 23.04.1998 US 64704; 23.04.1998 US 65158
(43) Date of publication of application: 15.09.1999
(73) Proprietor: Boston Scientific Limited, St. Michael, Barbados (BB)
(72) Inventor: BATES, James, S., Bloomington, IN 47404 (US); RILEY, James, W., Bloomington, IN 47401 (US)
(74) Representative: Lawrence, John
(86) International application number: PCT/US1998/020560
(87) International publication number: WO 1999/016365

(56) References cited:
- DE-A- 4 025 799
- DE-U- 8 707 516
- US-A- 2 556 783
- US-A- 5 030 201
- US-A- 5 658 296

## Description

### Technical Field

The invention relates generally to baskets for retrieving material in a body. More particularly, the invention relates to urology baskets having legs with a certain cross-section designed to retrieve kidney stones, urinary calculi, choteliths, or the like from within the body while causing less trauma to the body and enhancing stone capture.

### Background Information

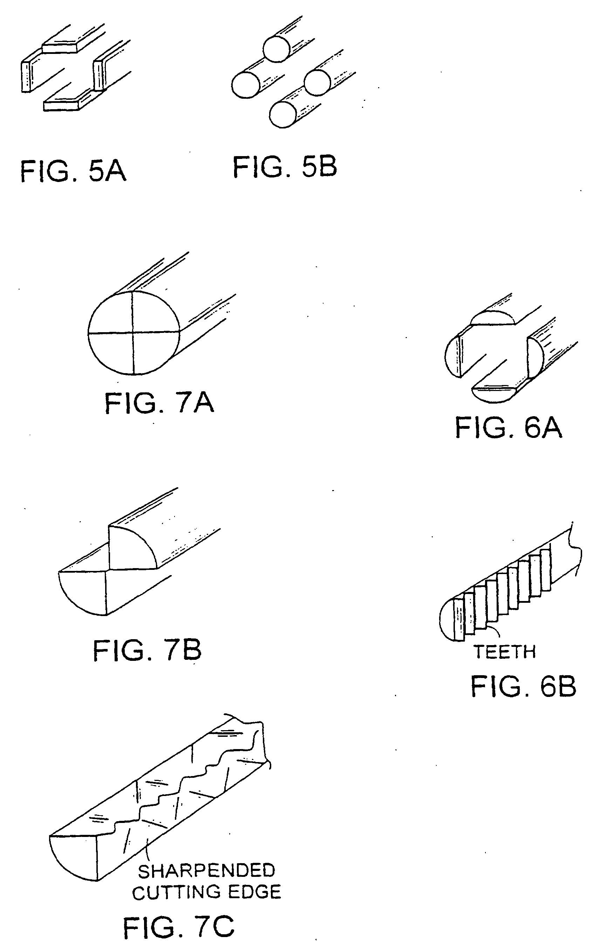

Baskets are used to retrieve various foreign or biological materials (e.g., kidney stones, urinary calculi, choleliths, etc.) from within a body. Baskets are used in the field of urology and other areas such as, for example, endoscopy. Existing retrieval baskets typically include a plurality of wires (or legs) which truncate at a base of the basket. It is known to form the legs of the basket from wire having a rectangular cross-section (FIG. 5A) or a round cross-section (FIG. 5B). The legs are joined by solder, weld, or mechanical means at the base of the basket and at its distal tip. At the base. the legs also are attached to a shaft coil (or wire). This coil is moved back and forth within a sheath or catheter by an actuation device (e.g., a proximal handle with a back-and-forth thumb-activated slider). By moving the coil forward with the actuation device, the legs are extended out of the distal end of the catheter and thus allowed to expand and form the basket shape beyond the distal end of the catheter. Moving the coil back causes the basket to retract into the catheter. Such a device is disclosed in DE-U-8 707 516 which has been used for the delincation in two-part form of claim 1.

### Summary of the Invention

In general, the advantages of basket legs having a rectangular cross-section include good dilatation force and good contact between the inner surface of the legs and the stone, and the disadvantages of such legs include sharp edges (i.e., the top two corners of the rectangle that contact the interior wall of the lumen in which the basket is disposed) and thus tissue trauma, inability to rotate the basket due to the danger of tissue trauma, and the difficulty of forming a helix-shaped basket structure when the basket is expanded. In general, the advantages and disadvantages of basket legs having a round (or circular) cross-section are the opposite of those encountered with legs of rectangular cross-section. That is, the advantages of legs of round or circular cross-section generally include round edges and thus reduced tissue trauma, ability to rotate the basket without danger of tissue trauma, and ability to form a helix-shaped basket structure, and the disadvantages of legs of round cross-section generally include less dilatation force and less contact between the inner surface of the legs and the stone being captured.

The invention relates to a medical retrieval basket having legs with a cross sectional shape designed to enhance the capture and retrieval of foreign materials or biological materials (e.g., kidney stones, urinary calculi, etc.) from within a body while causing less trauma to tissue. The two basic design objectives according to the invention are (1) to cause less trauma to body tissues and to the linings of lumens of the body in which the basket is placed and manipulated to accomplish the retrieval of material and (2) to enhance the material-capturing ability of the basket. One shape that meets the two basic design goals of the invention is a basket having legs with a D-shape in cross-section. Advantages of the D-shape, and all other shapes according to the invention, include less trauma to tissue, ability to rotate the basket without forming the legs in a helix configuration, improved stone purchase and contact, ability to use the basket for tissue biopsy because of its improved cutting ability, improved stone breaking and/or crushing ability, ability to reduce the overall size of the catheter basket device while maintaining or increasing the opening force of the basket, and improved dilatation force compared to a basket with legs of round cross-section while making the basket less tissue traumatic.

In accordance with the invention, a medical retrieval devices has a basket formed by three or more legs to retrieve foreign or biological material. At least one of the legs has at least two surfaces, namely, an inner surface and an outer surface. The outer surface is an atraumatic surface such as a curved surface. The atraumatic outer surface can include one or more radii. The inner surface can be flat such that the leg has a D-shaped cross section. Other shapes are possible for the inner surface including a pointed shape that enhances the basket's stone crushing or breaking ability. The inner surface, whatever its shape, can have a smooth

Surface, or it can have a rough surface (e.g. serrated, etched, toothed, etc.) for further enhancing the basket's ability to capture material such as stones and other calculi.

In one aspect, the invention involves a medical retrieval device, comprising:
a proximal handle;
a sheath including a proximal portion, a distal end, and a lumen, the sheath extending distally from the handle; and
a basket, the basket and the sheath moveable relative to each other to achieve a retracted position in which the basket is collapsed within the lumen of the sheath, and an extended position in which the basket extends from the distal end of the sheath, the basket comprising at least three wires, the wires joined at a distal end of the basket for capturing material when the basket is in the extended position, and for holding material when the basket is in the extended position, and for holding material when the basket is at least partially collapsed in the sheath, at least one of the wires comprising an inner surface and an outer surface, wherein at least a portion of said inner surface and an outer surface, wherein at least a portion of said inner surface is flat, and at least a portion of said outer surface is curved, and wherein the basket is positioned at the distal end of the device for capturing material in a body.

Embodiments of the invention can include the following features. For example, the leg with the at least two surfaces can have a cross section defined by the surfaces, and the cross section can comprise a D-shape defined by the surfaces wherein the curved surface comprises an outer surface and the other surface comprises an inner surface. In general, the curved surface can comprise an outer surface and the other surface can comprise an inner surface. The inner surface can comprise a flat surface such that the cross section comprises a D-shape, or the inner surface can be wedge-shaped and comprise at least one point. Also, the inner surface can comprise a surface that is rougher than the outer surface, and this rough inner surface can comprise, for example, a serrated surface, a toothed surface, or an etched surface. The curved surface can comprise more than one radius (e.g. a B-shape). The basket can have three or more legs.

In an embodiment of the invention the device includes an extractor for removable insertion into the body, the extractor comprising said basket so that the material can be retrieved from the body.

The captured material may be a calculus such as, for example, a kidney stone, a ureteral stone, a urethral stone, a urinary bladder stone, or a stone in the biliary tree such as a gallbladder stone or a bile duct stone. Also, the device may be arranged to break the material into two or more pieces with, for example, a sharpened inner surface.

The foregoing and other objects, aspects, features, and advantages of the invention will become more apparent from the following description and from the claims.

### Brief Description of the Drawings

In the drawings, like reference characters generally refer to the same parts throughout the different views. Also, the drawings are not necessarily to scale, emphasis instead generally being placed upon illustrating the principles of the invention.
FIG. 1 is a plan view of a surgical extractor with a handle at a proximal end and a retrieval basket at a distal end.
FIG. 2 is a perspective view of an expanded basket with D-shaped legs according to the invention.
FIG. 3 is a plan view of the distal/basket end of the extractor of FIG. 1.
FIG. 4 is an end view of the extractor showing the tip of the basket and the four legs extending therefrom.
FIGS. 5A and 5B are examples of legs of prior art baskets which include flat or rectangular cross-section legs (FIG. 5A) and round or circular cross-section legs (FIG. 5B).
FIG. 6A shows one embodiment of basket legs according to the invention, namely, D-shaped legs, the view being a cross section along "cross sections" in FIG. 4.
FIG. 6B shows an inner surface of one of the basket legs of FIG. 6A, where the inner surface is rough to further enhance capture.
FIGS. 7A and 7B together show another embodiment of basket legs according to the invention, namely, V-shaped legs with atraumatic rounded outer surfaces, the views being cross sections along "cross sections" in FIG. 4.
FIG. 7C shows an inner surface of one of the basket legs of FIGS. 7A and 7B.
FIGS. 8A-8E are various other possible cross-sectional shapes for the basket legs in accordance with the invention, the views being cross sections along "cross sections" in FIG. 4.

### Description

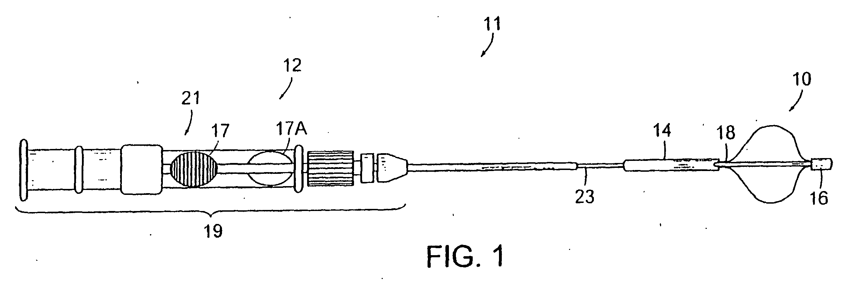

Referring to FIG. 1, a basket 10 for retrieving biological material or foreign material is attached to an actuation device 12 via a sheath or catheter 14. The basket 10 includes a plurality of legs (or wires) attached at a basket tip 16 and base 18. At the base 18, the legs are also attached to a shaft coil, cable, or wire 23 located in the sheath 14. This cable 23 is actuated by the actuation device 12 (e.g., a slider 21 on a proximal handle 19) to move the cable 23 and thus the basket legs, and the basket 10 thus is advanced and retracted by use of the actuation device 12.

More particularly, with continued reference to FIG. 1, the basket 10 typically is part of a distal end of an extractor 11 which an operator (e.g., a physician) introduces into a patient in a form in which the basket 10 is retracted. In the retracted state, the sheath 14 retains the basket 10 in a compact form until the extractor 11 is positioned proximate to material to be retrieved such as, for example, a kidney stone, a ureteral stone, urethral stone, a bladder stone, a gallbladder stone, cholelith or bile duct stone. The operator holding the handle 19 moves the slider 21 connected to the sheath 14 from the position 17A shown in phantom to the position 17. In the position 17A, the basket 10 is fully deployed out of the sheath 14, and it is fully retracted when the slider 21 is in the position 17. The basket 10 is shown fully deployed in FIG. 1. With the basket 10 fully deployed and extending out of the end of the sheath 14, the surrounding tissue is dilated and the basket 10 assumes a structure that can be manipulated over the material (e.g., a calculus). The operator then manipulates the retrieval basket 10 via the proximally extending control cable 23 connected to the wires or legs of the basket 10 and captures material in the basket 10. After capture, the sheath 14 advances distally and reduces the volume of the retrieval basket 10 until it contacts the entrapped material so the operator can withdraw the extractor 11 with the entrapped material.

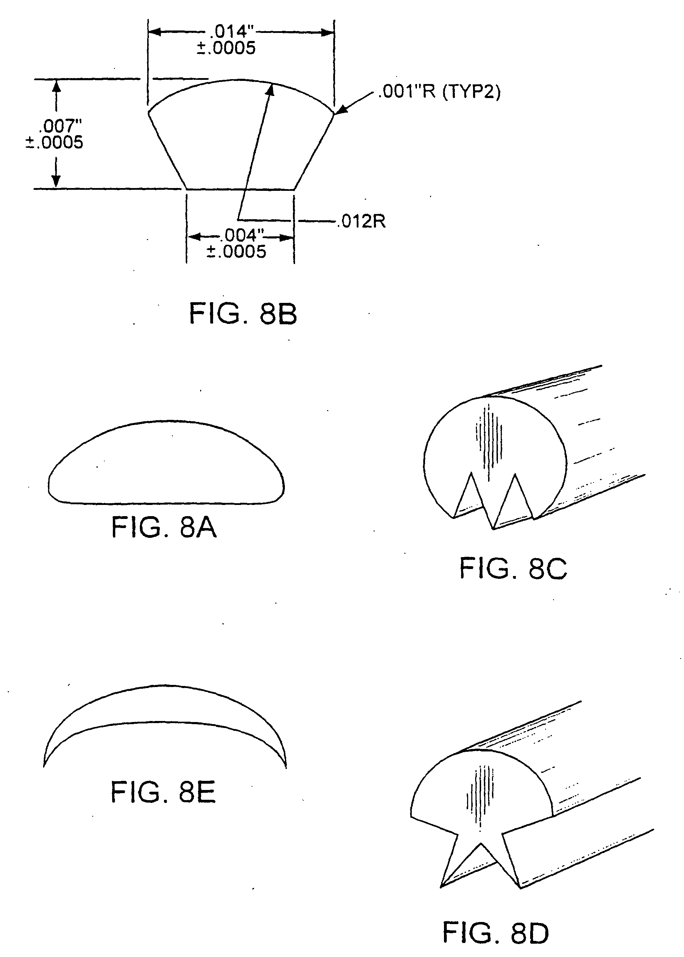

Known cross-sectional shapes for basket legs are rectangular (FIG. 5A) or round (FIG. 5B). However, in accordance with the invention and as shown in FIGS. 6 and 7, the basket 10 can have D-shaped legs (i.e., legs that each have a rounded outer surface and a flat inner surface) or V-shaped legs (i.e., legs that each have a rounded outer surface and an inner surface with a wedge shape and a point). Other leg shapes according to the invention are shown in FIG. 8. Whatever the particular leg shape, the shape will, according to the invention, (1) introduce less trauma and (2) enhance material capture. Also, whatever the particular leg shape, the legs can be drawn or ground or formed in a variety of other ways to achieve the shape. The legs can extend along a generally linear or helical path as described, for example, in U.S. Patent No. 5,658,296.

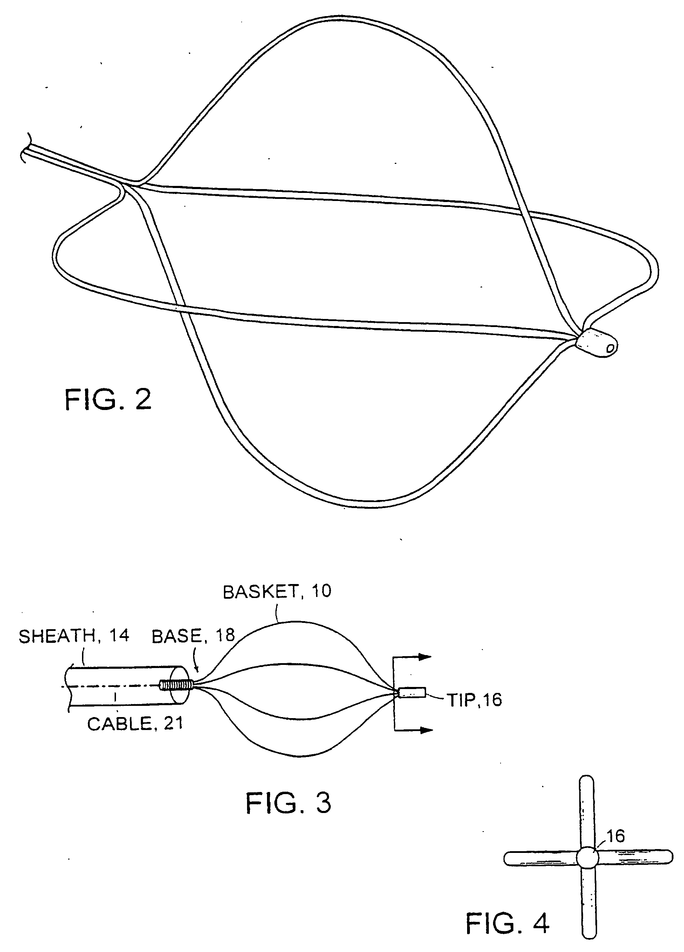

Referring to FIG. 2, the basket 10 of FIG. 1 is shown expanded. This particular basket has four D-shaped legs. In general, the basket 10 is formed by one or more legs, and it typically is formed by at least three legs. A simplified view of this basket is also shown in FIGS. 3 and 4. In those drawings, FIG. 3 shows just the distal end of the extractor 11, and FIG. 4 shows the legs of the basket 10 when viewed from the tip 16 down the length of the extractor 11.

Referring to FIGS. 6A and 6B, which like FIGS. 7A-7C and 8A-8E are cross sections taken generally along the cut labeled "cross sections" in FIG. 3, one disclosed embodiment of a basket according to the invention includes four D-shaped legs (FIG. 6A). The inner surface of one or more of these D-shaped legs can be rough so as to enhance the stone-capturing and holding ability of the basket. The roughness can be created in a variety of manners including serrations, teeth, etching, etc. on the inner surface(s). In FIG. 6B, teeth are shown on the inner surface of one of the D-shaped legs of FIG. 6A. Even without the rough inner surface, the D-shape, with its flat inner surface, enhances the ability of the basket to engage and hold stones. The outer surface of each of the D-shaped legs is rounded to form a smooth, atraumatic outer surface. The leg shape of FIG. 6A thus meets the two basic goals of any design in accordance with the invention, and that is less trauma and enhanced capture. The shape of the legs shown in FIG. 6A is a kind of hybrid between the known rectangular shaped legs (FIG. 5A) and the known round shaped legs (FIG. 5B), and the D-shaped legs of FIG. 6A generally have the advantages of each of these types of known legs without the disadvantages associated with the rectangular and round leg shapes. That is, the advantages of a basket with the D-shaped legs of the invention include increased dilatation force, better contact between the inner surface of the legs and the stone, atraumatic edges and thus reduced tissue trauma, ability to rotate the basket without danger of tissue trauma thereby increasing the ability to access and capture calculi disposed in difficult-to-reach locations, and ability to form easily a helix-shaped basket structure or a non-helix-shaped basket structure, and such a basket according to the invention lacks the disadvantages of sharp traumatic outer surface edges, inability to rotate the basket due to the danger of tissue trauma, difficulty in forming a helix-shaped basket structure, weak dilatation force, and poor contact between the inner surface of the legs and the stone being captured.

Although the outer surfaces of the basket legs are shown in FIG. 6A as being rounded, it is possible to utilize other shapes for the outer surface and still be within the scope of the invention. For example, in FIG. 6A, the rounded outer surface could be replaced with any curved surface (e.g., a B-shape). In general, any type of outer surface is possible as long as it furthers one of the two goals of the invention which is to reduce trauma to the body to something less than that provided by the generally traumatic two-comer shape of the outer surface of the conventional rectangular wire basket legs. In general, a curved outer surface will satisfy this design requirement of the invention.

Also, although the inner surfaces of the basket legs are shown in FIG. 6A as being flat, it is possible to utilize other shapes for the inner surface and still be within the scope of the invention. For example, in FIG. 6A, the flat inner surface could be replaced with a concave surface such that the D-shape becomes a crescent shape (FIG. 8E). In general, any type of inner surface is possible as long as it furthers the second of the two goals of the invention which is to enhance the capturing and holding ability of the basket over that of the generally poor performance of conventional round wire basket legs. Also, any type of inner surface according to the invention can be smooth or it can be rough as described above.

Some additional basket leg shapes according to the invention are shown in FIGS. 7A-7C and 8A-8E and are described below with reference to those drawings. In general, these, and the other specific shapes disclosed herein, are just some of the possible leg shapes according to the invention:

Referring to FIGS. 7A and 7B, another disclosed embodiment of a basket according to the invention includes four generally V-shaped or wedge-shaped legs. As with the D-shaped wires of FIGS. 6A and 6B, the inner surface of one or more of these legs can be rough so as to enhance the stone-capturing and holding ability of the basket, and the roughness can be created in a variety of manners including serrations, teeth, etching, etc. on the inner surface(s). In FIG. 7C, grooves are shown on the inner surface of one of the V-shaped legs of FIGS. 7A and 7B. Even without the rough inner surface, the V-shape, with its point enhances the ability of the basket to engage and hold stones and to break and/or crush the stones if desired. In fact, the point or ridge on the inner surface of these wedge-shaped legs can be sharpened to form a cutting edge that will easily and firmly grip into calculi when the basket is collapsed therearound and that can cut or bite into stones to break and/or crush them into two or more pieces. As with the D-shaped wire of FIGS. 6A and 6B. the outer surface of each of the V- or wedge-shaped legs is rounded to form a smooth, atraumatic outer surface. The leg shape of FIGS. 7A and 7B thus meets the two basic goals of any design in accordance with the invention, and that is less trauma and enhanced capture. Like the D-shaped legs of FIGS. 6A and 6B, the legs of FIGS. 7A-7C includes advantages such as increased dilatation force, better contact between the inner surface of the legs and the stone, atraumatic edges and thus reduced tissue trauma, ability to rotate the basket without danger of tissue trauma thereby increasing the ability to access and capture calculi disposed in difficult-to-reach locations, and ability to form easily a helix-shaped basket structure or a non-helix-shaped basket structure, and the legs of FIGS. 7A-7C have none of the disadvantages of known basket leg shapes such as sharp traumatic outer surface edges, inability to rotate the basket due to the danger of tissue trauma difficulty in forming a helix-shaped basket structure, weak dilatation force, and poor contact between the inner surface of the legs and the stone being captured.

As discussed above for the D-shaped legs of FIG. 6A, although the outer surfaces of the basket legs shown in FIGS. 7A-7C are rounded, it is possible to utilize other shapes for the outer surface and still be within the scope of the invention. The same is true for any of the other shapes according to the invention including those shown in FIGS. 8A-8E and discussed below with reference to those drawings. For example, in FIGS. 7A and 7B, the rounded outer surface could be replaced with any curved surface (e.g., a B-shape). Again, in general, any type of outer surface is possible as long as it furthers one of the two goals of the invention which is to reduce trauma to the body to something less than that provided by the generally traumatic two-corner shape of the outer surface of the conventional rectangular wire basket legs, and a curved outer surface satisfies this design requirement of the invention.

Also and as discussed above for the D-shaped legs of FIG. 6A, although the inner surfaces of the basket legs are shown in FIGS. 7A-7C as being V- or wedge-shaped, it is possible to utilize other shapes for the inner surface and still be within the scope of the invention. The same is true for any of the other shapes according to the invention including those shown in FIGS. 8A-8E and discussed below with reference to those drawings. Again, in general, any type of inner surface is possible as long as it furthers the second of the two goals of the invention which is to enhance the capturing and holding ability of the basket over that of the generally poor performance of conventional round wire basket legs. Also, any type of inner surface according to the invention can be smooth or it can be rough as described above.

Referring to FIGS. 8A-8D, other possible cross-sectional shapes for the basket legs according to the invention include a modified D-shape (FIG. 8A), a shape that is part round and part trapezoidal (FIG. 8B), and a variety of other shapes including, for example, a two-groove shape (FIG. 8C) and a two-prong shape (FIG. 8D).

The cross-sectional shape of any of the basket legs can vary along the length of the leg. Also, a single basket can have individual legs of different shape such as, for example, one D-shaped leg, one B-shaped leg, and a third leg with the shape of FIG. 8B. Further, the various shapes according to the inventions can be formed by the basket legs themselves (as shown) or by other means such as coatings including, for example, teflon on the outer surface and/or rubber on the inner surface.

As indicated previously, the inner surface of each of the basket legs can be smooth or rough. Also. the inner surface can be rough along the entire length of the basket leg or for only a portion of the basket leg. These are true whatever the shape of the legs and whether or not they vary in shape along their length. In fact, even conventional basket legs can benefit from this enhancement, and thus rough inner surfaces of basket legs generally are a part of this invention. That is, roughening all or a portion of one or more of the inner surfaces of one or more of the legs of a conventional basket or of a basket as described herein forms a part of this invention. A retrieval basket having all or a portion of one or more of the inner surfaces of one or more of the basket legs roughened in accordance with the invention provides the benefits of increased stone capture and retention. All or a portion of the inner surface of any leg of any basket can be made rough in accordance with this invention by a variety of means including forming serrations on the inner surface of the leg, forming teeth on the inner surface of the leg, etching the inner surface of the leg to make it pitted and rough, sand blasting the inner surface of the leg, and a variety of other known techniques.

Variations, modifications, and other implementations of what is described herein will occur to those of ordinary skill in the art without departing from the scope of the invention as claimed.

## Claims

1. A medical retrieval device, comprising:
a proximal handle (19);
a sheath (14) including a proximal portion, a distal end, and a lumen, the sheath (14) extending distally from the handle (19); and
a basket (10), the basket (10) and the sheath (14) moveable relative to each other to achieve a retracted position in which the basket (10) is collapsed within the lumen of the sheath (14), and an extended position in which the basket (10) extends from the distal end of the sheath (14), the basket (10) comprising wires joined at a distal end of the basket (10) for capturing material when the basket (10) is in the extended position, and for holding material when the basket (10) is at least partially collapsed in the sheath (14), at least one of the wires comprising an inner surface and an outer surface, wherein at least a portion of said inner surface is flat, and wherein the basket (10) is positioned at the distal end of the device for capturing material in a body, **characterised in that** the basket comprises at least three wires, and at least a portion of said outer surface is curved.

2. The device of claim 1 wherein a cross section of the at least one wire comprises a B-shape.

3. The device of claim 1 wherein a cross-section of the at least one wire comprises a V-shape.

4. The device of claim 1 wherein a cross section of the at least one wire comprises a D-shape.

5. The device of claim 1 wherein the flat portion of the inner surface comprises at least one point.

6. The device of claim 1 wherein the flat portion of the inner surface comprises a surface that is rougher than the outer surface.

7. The device of claim 1 wherein the flat portion of the inner surface is rough.

8. The device of claim 7 wherein the flat portion of the inner surface comprises a serrated surface.

9. The device of claim 7 wherein the flat portion of the inner surface comprises a toothed surface.

10. The device of claim 7 wherein the flat portion of the inner surface comprises an etched surface.

11. The device of claim 1 wherein the curved portion of the outer surface comprises more than one radius.

12. The device of claim 1 wherein a cross-section of the at least one wire is substantially trapezoidal.

13. The device of claim 1 wherein the inner surface has at least a portion adapted to contact material in a body, the portion of the inner surface comprising a rough surface.

14. The device of claim 1 wherein the outer surface comprises at least one curved surface.

15. The device of claim 4 wherein the flat portion of the inner surface is serrated.

16. The device of claim 2 wherein the flat portion of the inner surface comprises a serrated surface.

17. The device of claim 4 wherein the flat portion of the inner surface comprises a toothed surface.

18. The device of claim 4 wherein the flat portion of the inner surface comprises an etched surface.

19. The device of any of the preceding claims inclding an extractor (11) for removable insertion into the body, the extractor comprising (11) said basket (10) so that the material can be retrieved from the body.

20. The device of any of the preceding claims wherein the material is a calculus.

21. The device of any of the preceding claims wherein the material is a kidney stone.

22. The device of any one of claims 1 to 20 wherein the material is a ureteral stone.

23. The device of any one of claims 1 to 20 wherein the material is a bladder stone.

24. The device of any one of claims 1 to 20 wherein the material is a stone in the biliary tree.

25. The device of any of the preceding claims wherein the material is broken into two or more pieces by the basket (10).

26. The device of claim 4 wherein the radius of the outer surface is substantially 0.012 inches (0.305 mm).

27. The device of claim 4 wherein the flat surface of the cross-section is 0.004 ± 0.0005 inches (0.102 ± 0.0127 mm) in length.

28. The device of claim 4 wherein the radius of the curved surface is substantially 0.012 inches (0.305 mm) and the flat surface of the cross-section is 0.004 inches ± 0.0005 inches (0.102 ± 0.0127 mm) in length.

## Patentansprüche

1. Medizinische Fangeinrichtung, die aufweist:
einen proximalen Griff (19);
einen Mantel (14), der einen proximalen Bereich, ein distales Ende und ein Lumen aufweist, wobei sich der Mantel (14) distal vom Griff (19) erstreckt; und
ein Fangkörbchen (10), wobei das Fangkörbchen (10) und der Mantel (14) in bezug aufeinander bewegbar sind, um eine Zurückziehposition zu erreichen, bei der das Körbchen (10) innerhalb des Lumens des Mantels (14) zusammenklappt, und einer erstreckten Position, bei der sich das Körbchen (10) vom distalen Ende des Mantels (14) erstreckt, wobei das Körbchen (10) Drähte aufweist, welche an einem distalen Ende des Körbchens (10) verbunden sind, um Material einzufangen, wenn das Körbchen (10) in der ausgebreiteten Position ist, und um das Material zu halten, wenn das Körbchen (10) zumindest teilweise im Mantel (14) zusammengeklappt ist, wobei zumindest einer der Drähte eine Innenfläche und eine Außenfläche aufweist, wobei zumindest ein Bereich der Innenfläche flach ist und wobei das Körbchen (10) am distalen Ende der Einrichtung angeordnet ist, um Material in einem Körper einzufangen,
**dadurch gekennzeichnet, dass** das Körbchen zumindest drei Drähte aufweist und zumindest ein Bereich der Außenfläche gekrümmt ist.

2. Einrichtung nach Anspruch 1, wobei ein Querschnitt des zumindest einen Drahts eine B-Form aufweist.

3. Einrichtung nach Anspruch 1, wobei ein Querschnitt des zumindest einen Drahts eine V-Form aufweist.

4. Einrichtung nach Anspruch 1, wobei ein Querschnitt des zumindest einen Drahts eine D-Form aufweist.

5. Einrichtung nach Anspruch 1, wobei der flache Bereich der Innenfläche zumindest eine Erhebung aufweist.

6. Einrichtung nach Anspruch 1, wobei der flache Bereich der Innenfläche eine Fläche aufweist, die rauer ist als die Außenfläche.

7. Einrichtung nach Anspruch 1, wobei der flache Bereich der Innenfläche rau ist.

8. Einrichtung nach Anspruch 7, wobei der flache Bereich der Innenfläche eine gezackte Fläche aufweist.

9. Einrichtung nach Anspruch 7, wobei der flache Bereich der Innenfläche eine gezahnte Fläche aufweist.

10. Einrichtung nach Anspruch 7, wobei der flache Bereich der Innenfläche eine geätzte Fläche aufweist.

11. Einrichtung nach Anspruch 1, wobei der gekrümmte Bereich der Außenfläche mehr als einen Radius aufweist.

12. Einrichtung nach Anspruch 1, wobei ein Querschnitt des zumindest einen Drahts im Wesentlichen trapezförmig ist.

13. Einrichtung nach Anspruch 1, wobei die Innenfläche zumindest einen Bereich hat, der ausgebildet ist, Material in einem Körper zu kontaktieren, wobei der Bereich der Innenfläche eine raue Fläche aufweist.

14. Einrichtung nach Anspruch 1, wobei die Außenfläche zumindest eine gekrümmte Fläche aufweist.

15. Einrichtung nach Anspruch 4, wobei der flache Bereich der Innenfläche gezackt ist.

16. Einrichtung nach Anspruch 2, wobei der flache Bereich der Innenfläche eine gezackte Fläche aufweist.

17. Einrichtung nach Anspruch 4, wobei der flache Bereich der Innenfläche eine gezahnte Fläche aufweist.

18. Einrichtung nach Anspruch 4, wobei der flache Bereich der Innenfläche eine geätzte Fläche aufweist.

19. Einrichtung nach einem der vorhergehenden Ansprüche, welche eine Extraktionszange (11) aufweist, um diese in den Körper entfernbar einzufügen, wobei die Extraktionszange (11) das Körbchen (10) aufweist, so dass das Material vom Körper aufgefangen werden kann.

20. Einrichtung nach einem der vorhergehenden Ansprüche, wobei das Material ein Gallenstein ist.

21. Einrichtung nach einem der vorhergehenden Ansprüche, wobei das Material ein Nierenstein ist.

22. Einrichtung nach einem der Ansprüche 21, wobei das Material ein Urethralstein ist.

23. Einrichtung nach einem der Ansprüche 1 bis 20, wobei das Material ein Blasenstein ist.

24. Einrichtung nach einem der Ansprüche 1 bis 20, wobei das Material ein Stein im Gallengang ist.

25. Einrichtung nach einem der vorhergehenden Ansprüche, wobei das Material in zwei oder mehrere Stücke durch das Körbchen (10) gebrochen wird.

26. Einrichtung nach Anspruch 4, wobei der Radius der Außenfläche im Wesentlichen 0,012 Inches (0,305 mm) beträgt.

27. Einrichtung nach Anspruch 4, wobei die flache Fläche des Querschnitts 0,004 ± 0,0005 Inches (0,102 ± 0,0127 mm) lang ist.

28. Einrichtung nach Anspruch 4, wobei der Radius der gekrümmten Fläche im Wesentlichen 0,012 Inches (0,305 mm) ist und die flache Fläche des Querschnitts 0,004 Inches ± 0,0005 Inches (0,102 ± 0,0127 mm) lang ist.

## Revendications

1. Dispositif d'extraction médical, comprenant :
un manche proximal (19) ;
une gaine (14) comprenant une partie proximale, une extrémité distale, et une lumière, la gaine (14) s'étendant de manière distale à partir du manche (19) ; et
un panier (10), le panier (10) et la gaine (14) étant mobiles l'un par rapport à l'autre pour obtenir une position rétractée dans laquelle le panier (10) est replié à l'intérieur de la lumière de la gaine (14), et une position étendue dans laquelle le panier (10) s'étend à partir de l'extrémité distale de la gaine (14), le panier (10) comprenant des fils assemblés au niveau d'une extrémité distale du panier (10) pour saisir la matière lorsque le panier (10) est dans la position étendue, et pour supporter la matière lorsque le panier (10) est au moins partiellement replié dans la gaine (14), au moins l'un des fils comprenant une surface interne et une surface externe, dans lequel au moins une partie de ladite surface interne est plate, et dans lequel le panier (10) est positionné à l'extrémité distale du dispositif pour saisir la matière dans un corps, **caractérisé en ce que** le panier comprend au moins trois fils, et **en ce qu'**au moins une partie de ladite surface externe est incurvée.

2. Dispositif selon la revendication 1 **caractérisé en ce qu'**une section transversale du au moins un fil comprend une forme de B.

3. Dispositif selon la revendication 1, **caractérisé en ce qu'**une section transversale du au moins un fil comprend une forme de V.

4. Dispositif selon la revendication 1, **caractérisé en ce qu'**une section transversale du au moins un fil comprend une forme de D.

5. Dispositif selon la revendication 1, **caractérisé en ce que** la partie plate de la surface interne comprend au moins un point.

6. Dispositif selon la revendication 1, **caractérisé en ce que** la partie plate de la surface interne comprend une surface qui est plus rugueuse que la surface externe.

7. Dispositif selon la revendication 1, **caractérisé en ce que** la partie plate de la surface interne est rugueuse.

8. Dispositif selon la revendication 7, **caractérisé en ce que** la partie plate de la surface interne comprend une surface dentelée.

9. Dispositif selon la revendication 7, **caractérisé en ce que** la partie plate de la surface interne comprend une surface dentée.

10. Dispositif selon la revendication 7, **caractérisé en ce que** la partie plate de la surface interne comprend une surface gravée.

11. Dispositif selon la revendication 1, **caractérisé en ce que** la partie incurvée de la surface externe comprend plus d'un rayon.

12. Dispositif selon la revendication 1, **caractérisé en ce qu'**une section transversale du au moins un fil est sensiblement trapézoïdale.

13. Dispositif selon la revendication 1, **caractérisé en ce que** la surface interne a au moins une partie adaptée pour être en contact avec la matière dans le corps, la partie de la surface interne comprenant une surface rugueuse.

14. Dispositif selon la revendication 1, **caractérisé en ce que** la surface externe comprend au moins une surface incurvée.

15. Dispositif selon la revendication 4, **caractérisé en ce que** la partie plate de la surface interne est dentelée.

16. Dispositif selon la revendication 2, **caractérisé en ce** la partie plate de la surface interne comprend une surface dentelée.

17. Dispositif selon la revendication 4, **caractérisé en ce** la partie plate de la surface interne comprend une surface dentée.

18. Dispositif selon la revendication 4, **caractérisé en ce** la partie plate de la surface interne comprend une surface gravée.

19. Dispositif selon l'une quelconque des revendications précédentes, **caractérisé en ce qu'**il comprend un extracteur (11) pour l'insertion amovible dans le corps, l'extracteur comprenant (11) ledit panier (10) de sorte que la matière peut être extraite du corps.

20. Dispositif selon l'une quelconque des revendications précédentes, **caractérisé en ce que** la matière est un calcul.

21. Dispositif selon l'une quelconque des revendications précédentes, **caractérisé en ce que** la matière est un calcul rénal.

22. Dispositif selon l'une quelconque des revendications 1 à 20, **caractérisé en ce que** la matière est un calcul de l'uretère.

23. Dispositif selon l'une quelconque des revendications 1 à 20, **caractérisé en ce que** la matière est un calcul de la vessie.

24. Dispositif selon l'une quelconque des revendications 1 à 20, **caractérisé en ce que** la matière est un calcul dans l'arbre biliaire.

25. Dispositif selon l'une quelconque des revendications précédentes, **caractérisé en ce que** la matière est cassée en deux morceaux ou plus par le panier (10).

26. Dispositif selon la revendication 4, **caractérisé en ce** le rayon de la surface externe est sensiblement de 0,012 pouces (0,305 mm).

27. Dispositif selon la revendication 4, **caractérisé en ce** la surface plate de la section transversale mesure 0,004 ± 0,0005 pouces (0,102 ± 0,0127 mm) de longueur.

28. Dispositif selon la revendication 4, **caractérisé en ce** le rayon de la surface incurvée est sensiblement de 0,012 pouces (0,305 mm) et en ce que la surface plate de la section transversale mesure 0,004 pouces ± 0,0005 pouces (0,102 ± 0,0127 mm) de longueur.
